# EUROPEAN PATENT APPLICATION

(11) **EP 4 571 288 A1**
(43) Date of publication of application: **18.06.2025**
(21) Application number: 24216238.6
(22) Date of filing: 28.11.2024
(51) Int. Cl.: G01N 15/01, G01N 15/1434

(54) **SYSTEM AND METHOD FOR MEASURING ADHESION OF RED BLOOD CELLS IN MICROFLUIDIC CHANNNELS**

(30) Priority: 15.12.2023 US 202318542154
(71) Applicant: Honeywell International Inc., Charlotte, NC 28202 (US); Emory University, Atlanta, GA 30322 (US)
(72) Inventor: BROWN, Andy W., Charlotte, 28202 (US); ZAKARIA, Ryadh A., Charlotte, 28202 (US); SHAFAI, Moin S., Charlotte, 28202 (US); LAM, Wilbur, Charlotte, 28202 (US); FIBBEN, Kirby, Charlotte, 28202 (US); WILLIAMS, Evelyn Kendall, Charlotte, 28202 (US); EVANS, Erica, Charlotte, 28202 (US); SHEEHAN, Vivien, Charlotte, 28202 (US)
(74) Representative: Haseltine Lake Kempner LLP

(57) **Abstract**

A system and a method for measuring adhesion of blood cells in microfluidic channels are disclosed. The system includes a device having one or more microfluidic channels configured to adhere a plurality of diseased red blood cells (RBCs) of a blood sample on an interior surface of the one or more microfluidic channels. Further, the system includes at least one imager configured to generate one or more digital holography images or videos of the plurality of diseased RBCs adhered to the interior surface of the one or more microfluidic channels. Further, at least one processor is operationally coupled to the at least one imager and configured to receive the one or more digital holography images or videos and analyze the generated one or more digital holography images or videos to quantify adhesion of the plurality of diseased RBCs to the interior surface of the one or more microfluidic channels.

## Description

### TECHNOLOGICAL FIELD

Example embodiments of the present disclosure relate generally to a diagnostic device, and more particularly, to systems, apparatuses, and methods that utilize microfluidic channels and an inline digital holography technique to determine severity of a hematologic disease.

### BACKGROUND

Sickle cell disease (SCD) is a group of inherited red blood cell disorders that affects the shape of red blood cells, which carry oxygen to all parts of the body. SCD requires early diagnosis and constant monitoring and treatment throughout a patient's lifespan. About 3 million people worldwide suffer from SCD, mostly in Africa, India and the Middle East, with an estimated 100,000 affected in the U.S., according to the Centers for Disease Control and Prevention. SCD affects 1 in 375 African American newborns born in the U.S. Early diagnosis through newborn screening, followed by simple interventions, has dramatically reduced SCD related mortality in the US. More than 800 children are born with SCD every day in Africa, and more than half of the children die in childhood due to lack of diagnosis and early treatment. Effective treatment of SCD still remains a challenge in preventing childhood and adult mortality, especially in the developing world due to requirements of skilled personnel and the high cost of currently available modalities. Frequent SCD severity monitoring presents insurmountable challenges due to a lack of objective measures of the disease state and reliance on highly subjective patient-reported symptoms like pain.

The inventors have identified numerous areas of improvement in the existing technologies and processes, which are the subjects of embodiments described herein. Through applied effort, ingenuity, and innovation, many of these deficiencies, challenges, and problems have been solved by developing solutions that are included in embodiments of the present disclosure, some examples of which are described in detail herein.

### BRIEF SUMMARY

The following presents a summary of some example embodiments to provide a basic understanding of some aspects of the present disclosure. This summary is not an extensive overview and is intended to neither identify key or critical elements nor delineate the scope of such elements. It will also be appreciated that the scope of the disclosure encompasses many potential embodiments in addition to those here summarized, some of which will be further described in the detailed description that is presented later.

In an example embodiment, a system is disclosed. The system includes a device having one or more microfluidic channels configured to selectively adhere a plurality of diseased red blood cells (RBCs) of a blood sample on an interior surface of the one or more microfluidic channels. The system further includes at least one imager configured to generate one or more digital holography images or videos of the plurality of diseased RBCs adhered to the interior surface of the one or more microfluidic channels. The system further includes at least one processor operationally coupled to the at least one imager. The at least one processor is configured to receive the one or more digital holography images or videos, and analyze the generated one or more digital holography images or videos to quantify adhesion of the plurality of diseased RBCs to the interior surface of the one or more microfluidic channels.

In some embodiments, the interior surface of the one or more microfluidic channels is coated with a coating to cause the plurality of diseased RBCs to selectively adhere to the interior surface of the one or more microfluidic channels. In some embodiments, the coating comprises at least one of Laminin or p-selectin.

In some embodiments, the one or more microfluidic channels comprises at least two inlets and an outlet. Further, at least one inlet of the at least two inlets receives the blood sample and the outlet allows to discharge the plurality of diseased RBCs that are not adhered to the interior surface of the one or more microfluidic channels.

In some embodiments, the at least one imager uses a lensless in-line digital holography configuration to generate the one or more digital holography images or videos.

In some embodiments, the at least one processor is configured to analyze the generated one or more digital holography images or videos using an Artificial Intelligence/Machine Learning (AI/ML) module to determine the number of adhered diseased RBCs on the interior surface of the one or more microfluidic channels.

In some embodiments, a quantification of the plurality of diseased RBCs adhered is configured to generate a score or relative indicator of a user's health status and/or indicate an efficacy of a medical treatment. In some embodiments, the plurality of diseased RBCs are unstained, untagged, and unmodified.

In some embodiments, at least one user device is configured to receive the quantified adhesion of the plurality of diseased RBCs to display a report related to the user's health status.

In another example embodiment, a method is disclosed. The method comprises steps of selectively adhering a plurality of diseased red blood cells (RBCs) of a blood sample on an interior surface of one or more microfluidic channels; generating, via at least one imager, one or more digital holography images or videos of the plurality of diseased RBCs adhered to the interior surface of the one or more microfluidic channels; and analyzing, via at least one processor, the generated one or more digital holography images or videos to quantify adhesion of the plurality of diseased RBCs to the interior surface of the one or more microfluidic channels.

In some embodiments, the method further includes analyzing, via the at least one processor, the generated one or more digital holography images or videos using an Artificial Intelligence/Machine Learning (AI/ML) module to determine a number of adhered RBCs of the plurality of diseased RBCs on the interior surface of the one or more microfluidic channels.

In some embodiments, the method further comprises transmitting to at least one user device a report indicating the user's health status based at least on the quantified adhesion of the plurality of diseased RBCs.

The above summary is provided merely for purposes of summarizing some example embodiments to provide a basic understanding of some aspects of the present disclosure. Accordingly, it will be appreciated that the above-described embodiments are merely examples and should not be construed to narrow the scope or spirit of the present disclosure in any way. It will be appreciated that the scope of the present disclosure encompasses many potential embodiments in addition to those here summarized, some of which will be further described below.

### BRIEF DESCRIPTION OF THE DRAWINGS

Having thus described certain example embodiments of the present disclosure in general terms, reference will hereinafter be made to the accompanying drawings, which are not necessarily drawn to scale, and wherein:
FIG. 1 illustrates a block diagram of a system to determine severity of a hematologic disease in accordance with an example embodiment of the present disclosure;
FIG. 2A illustrates a perspective view of an imaging device for determining severity of the hematologic disease in accordance with an example embodiment of the present disclosure;
FIG. 2B illustrates an enlarged view of the imaging device in accordance with an example embodiment of the present disclosure;
FIG. 3 illustrates a device with microfluidic channel having multiple dimensions at various points in accordance with an example embodiment of the present disclosure;
FIG. 4A illustrates an example holographic microscopy image with cell counting area in accordance with an example embodiment of the present disclosure;
FIG. 4B illustrates a binarized image with diseased red blood cells adhering to interior surface of one or more microfluidic channels in accordance with an example embodiment of the present disclosure;
FIG. 5 illustrates a flowchart of a method to determine severity of the hematologic disease i.e., sickle cell disease in accordance with an example embodiment of the present disclosure; and
FIG. 6 illustrates an exemplary scenario of the system to determine severity of the sickle cell disease in accordance with an example embodiment of the present disclosure.

### DETAILED DESCRIPTION

Some embodiments will now be described more fully hereinafter with reference to the accompanying drawings, in which some, but not all, embodiments of the present disclosure are shown. Indeed, various embodiments may be embodied in many different forms and should not be construed as limited to the embodiments set forth herein; rather, these embodiments are provided so that this disclosure will satisfy applicable legal requirements.

The components illustrated in the figures represent components that may or may not be present in various embodiments of the present disclosure described herein such that embodiments may include fewer or more components than those shown in the figures while not departing from the scope of the present disclosure. Some components may be omitted from one or more figures or shown in dashed line for visibility of the underlying components.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in various embodiments," "in one embodiment," "according to one embodiment," "in some embodiments," and the like generally mean that the particular feature, structure, or characteristic following the phrase may be included in at least one embodiment of the present disclosure and may be included in more than one embodiment of the present disclosure (importantly, such phrases do not necessarily refer to the same embodiment).

The word "example" or "exemplary" is used herein to mean "serving as an example, instance, or illustration." Any implementation described herein as "exemplary" is not necessarily to be construed as preferred or advantageous over other implementations.

If the specification states a component or feature "may," "can," "could," "should," "would," "preferably," "possibly," "typically," "optionally," "for example," "often," or "might" (or other such language) be included or have a characteristic, that a specific component or feature is not required to be included or to have the characteristic. Such a component or feature may be optionally included in some embodiments or it may be excluded.

The present disclosure provides various embodiments of systems, devices, and methods to measure adhesion of red blood cells in microfluidic channels. Embodiments may adhere a plurality of diseased red blood cells (RBCs) of a blood sample (or red cell suspension) on an interior surface of a microfluidic channel. Embodiments may generate one or more digital holography images or videos of the plurality of diseased RBCs adhered to the interior surface of the microfluidic channel. Embodiments may analyze the generated one or more digital holography images or videos to quantify adhesion of the plurality of RBCs to the interior surface of the microfluidic channels. Thereafter, a report related to user's health status is generated to show severity of a hematologic disease i.e., sickle cell disease in the plurality of RBCs.

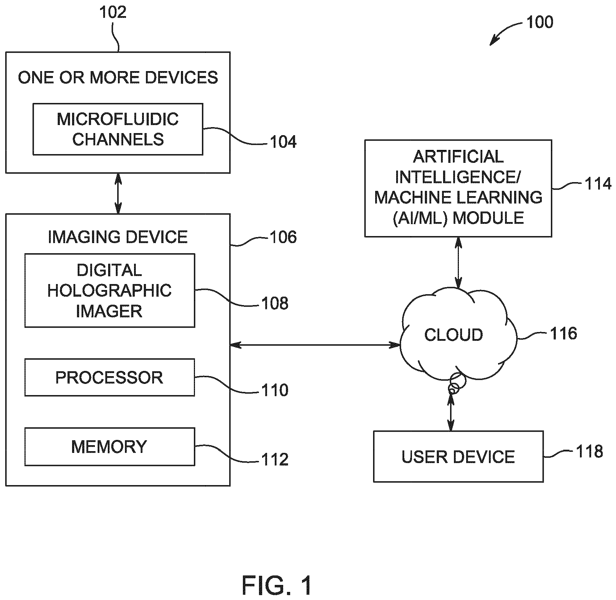

FIG. 1 illustrates a block diagram of a system 100 to determine severity of a hematologic disease in accordance with an example embodiment of the present disclosure.

The system 100 may comprise one or more devices 102 with microfluidic channels 104, and at least one imaging device 106. The at least one imaging device 106 may include at least one imager 108 and at least one processor 110 operated on stored instructions in a memory 112. The one or more devices 102 with microfluidic channels 104 may be configured to adhere a plurality of diseased red blood cells (RBCs) of a blood sample on an interior surface (not shown) of the one or more microfluidic channels 104. Hereinafter, the one or more devices 102 or a device 102 may be used interchangeably. In some embodiments, the device 102 may correspond to an adhesion device. The interior surface of the one or more microfluidic channels 104 may be coated with a coating to cause the plurality of diseased RBCs to selectively adhere to the interior surface of the one or more microfluidic channels 104. In some embodiments, the interior surface of the one or more microfluidic channels 104 may be coated with Laminin or p-selectin. In some example embodiments, various other coating options may exist depending on which pharma medication is being evaluated for efficacy, without departing from the scope of the disclosure. In some example embodiments, the coating may correspond to a protein coating.

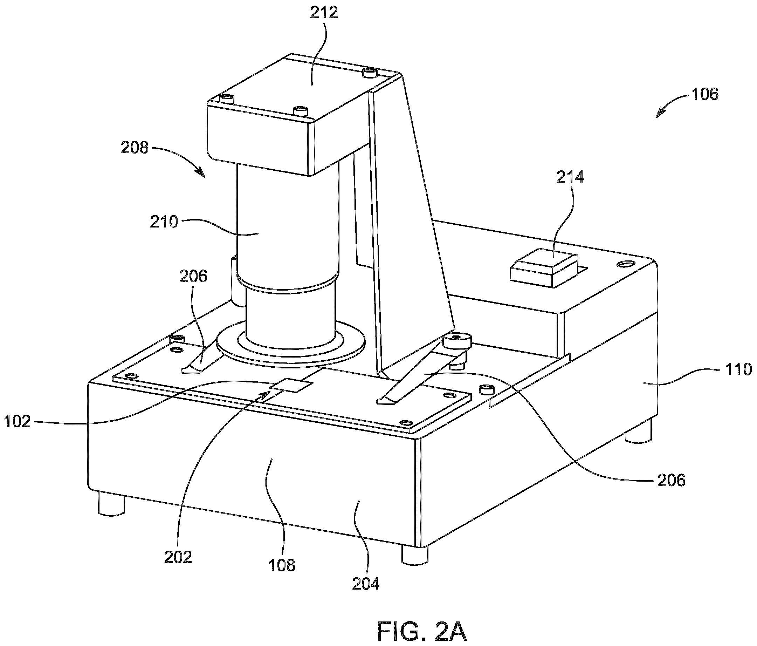

In some embodiments, the at least one imager 108 may be configured to generate one or more digital holography images or videos of the plurality of RBCs in the one or more microfluidic channels 104. It may be noted that the plurality of diseased RBCs may be selectively adhered to the interior surface of the one or more microfluidic channels 104. Further, the at least one imager 108 may be referred to as a digital holographic imager. In some embodiments, the at least one imager 108 may use a lensless in-line digital holography configuration to generate the one or more digital holography images or videos. Further, the at least one processor 110 may be operationally coupled to the at least one imager 108. The at least one processor 110 may be configured to receive the one or more digital holography images or videos. The at least one processor 110 may be configured to analyze the generated one or more digital holography images or videos to quantify adhesion of the plurality of RBCs to the interior surface of the one or more microfluidic channels 104. It may be noted that the plurality of RBCs may be unstained, untagged, or unmodified. The at least one processor 110 may be configured to analyze the generated one or more digital holography images or videos using Artificial Intelligence/Machine Learning (AI/ML) techniques to count the plurality of diseased RBCs adhered on the interior surface of the one or more microfluidic channels 104. The detailed working of the at least one processor 110 may be described in greater detail in conjunction with FIGS. 2A-4B.

In some embodiments, the at least one processor 110 may include suitable logic, circuitry, and/or interfaces that are operable to execute one or more instructions stored in the memory to perform predetermined operations. In one embodiment, the at least one processor 110 may be configured to decode and execute any instructions received from one or more other electronic devices or server(s). The at least one processor 110 may be configured to execute one or more computer-readable program instructions, such as program instructions to carry out any of the functions described in this description. Further, the processor may be implemented using one or more processor technologies known in the art. Examples of the processor include, but are not limited to, one or more general purpose processors (e.g., INTEL^{®} or Advanced Micro Devices^{®} (AMD) microprocessors) and/or one or more special purpose processors (e.g., digital signal processors or Xilinx^{®} System On Chip (SOC) Field Programmable Gate Array (FPGA) processor).

Further, the memory 112 may be communicatively coupled to the at least one processor 110. Further, the memory 112 may be configured to store a set of instructions and data executed by the one or more processors 110. Further, the memory 112 may include the one or more instructions that are executable by the one or more processor 110 to perform specific operations. It is apparent to a person with ordinary skill in the art that the one or more instructions stored in the memory 112 enable the hardware of the system 100 to perform the predetermined operations. Some of the commonly known memory implementations include, but are not limited to, fixed (hard) drives, magnetic tape, floppy diskettes, optical disks, Compact Disc Read-Only Memories (CD-ROMs), and magneto-optical disks, semiconductor memories, such as ROMs, Random Access Memories (RAMs), Programmable Read-Only Memories (PROMs), Erasable PROMs (EPROMs), Electrically Erasable PROMs (EEPROMs), flash memory, magnetic or optical cards, or other type of media/machine-readable medium suitable for storing electronic instructions.

Further, the system 100 may comprise an Artificial Intelligence/Machine Learning (AI/ML) module 114 communicatively coupled to the at least one processor 110 via a cloud 116. The at least one processor 110 along with the AI/ML module 114 may analyze the generated one or more digital holography images or videos to quantify adhesion of the plurality of RBCs to the interior surface of the one or more microfluidic channels 104. Such quantification of the plurality of diseased RBCs may be configured to generate a score or relative indicator of a user's health status or indicate an efficacy of a medical treatment of the user based on a severity of a hematologic disease in the plurality of RBCs. In some embodiments, the hematologic disease may correspond to diseases relating to blood cells or hemoglobin, such as, RBCs, White blood cells, Plasma, Platelet, etc. In some embodiments, the AI/ML module 114 may be configured to detect and classify each cell. Such classification may ensure that the detected cells are the plurality of RBCs and not some other cell type or dust/debris. In some alternate embodiments, the AI/ML module 114 may be integrated within the at least one processor 110 to analyze the generated one or more digital holography images or videos without the need for remotely monitoring, via the cloud 116.

In some embodiments, the cloud 116 may facilitate a communication link between the AI/ML module and the at least one processor 110. It may be noted that the cloud 116 may be referred as a network interface that facilitates a communication link among the other components of the system 100. Further, the cloud 116 may be a wireless network and/or a wired network. The cloud 116 may be implemented using one or more communication techniques. The one or more communication techniques may be Radio waves, Wi-Fi, Bluetooth, ZigBee, Z-wave and other communication techniques, known in the art.

The system 100 may further include at least one user device 118 that may be configured to receive the quantified adhesion of the plurality of RBCs to display a report related to user's health status. In some embodiments, the at least one user device 118 may be separate and remote from the system 100 and the system 100 may communicate with the remote user device. In some embodiments, the at least one user device 118 may be wired to the at least one processor 110 or may be coupled, via the cloud 116. In some embodiments, the at least one user device 118 may include a wired or wireless devices operationally coupled to the system 100. For example, a desktop or laptop computer, any tablet, smart phone, or other handheld devices.

Further, the system 100 may include an input/output circuitry (not shown) that may enable a user to communicate or interface with the system 100 via the at least one user device 118. In some example embodiments, the at least one user device 118 may include a control room computer system or other portable electronic devices. It may be noted that the input/output circuitry may act as a medium transmit input from the at least one user device 118 to and from the system 100. In some embodiments, the input/output circuitry may refer to hardware and software components that facilitate the exchange of information between the user and the system 100. In one example embodiment, the system 100 may include a graphical user interface (GUI) (not shown) as an input circuitry to allow the user to input data via the at least one user device 118. The input/output circuitry may include various input devices such as keyboards, barcode scanners, GUI for the user to provide data and various output devices such as displays, printers for the user to receive data.

In some alternate embodiments, the system 100 may include a communication circuitry (not shown). The communication circuitry may allow the at least one imaging device 106 and the at least one user device 118 to exchange data or information with other systems. Further, the communication circuitry may include network interfaces, protocols, and software modules responsible for sending and receiving data or information. In some embodiments, the communication circuitry may include Ethernet ports, Wi-Fi adapters, or communication protocols like Hypertext transfer protocol (HTTP) or Message Queuing Telemetry Transport (MQTT) for connecting with other systems. The communication circuitry may allow the system 100 to stay up-to-date and accurately track levels of adhesion blood sample/cells.

It will be apparent to one skilled in the art the above-mentioned components of the system 100 have been provided only for illustration purposes, without departing from the scope of the disclosure.

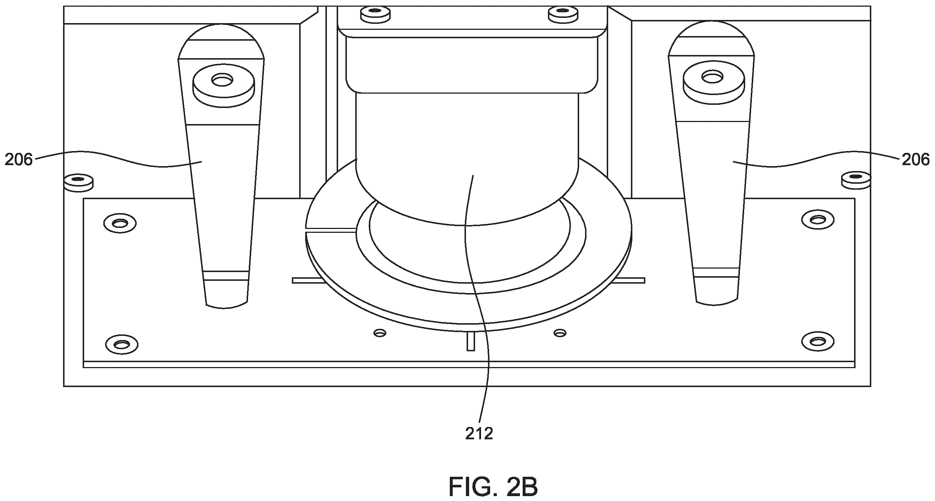

FIG. 2A illustrates a perspective view of the at least one imaging device 106 for determining severity of the hematologic disease in accordance with an example embodiment of the present disclosure. FIG. 2B illustrates an enlarged view of the at least one imaging device 106 in accordance with an example embodiment of the present disclosure. FIGS. 2A-2B are described in conjunction with FIG. 1.

The at least one imaging device 106 may comprise a cross hair 202 positioned over a housing 204. In some embodiments, the one or more microfluidic channels 104 of the device 102 may be utilized by the at least one imaging device 106 to determine severity of the hematologic disease. The one or more microfluidic channels 104 may be placed on the cross hair 202. The one or more microfluidic channels 104 may be positioned firmly on the cross hair 202 using one or more clamps 206. In some embodiments, the one or more clamps 206 may correspond to metal plates that may be rotatably fixed at one end and free at other end. The free end may slide onto the one or more microfluidic channels 104 once placed over the housing 204. Further, the one or more microfluidic channels 104 placed on the cross hair 202, may be configured to selectively adhere a plurality of diseased red blood cells (RBCs) of a blood sample on an interior surface (not shown) of the one or more microfluidic channels 104. In some embodiments, the interior surface of the one or more microfluidic channels 104 may be coated to cause the plurality of diseased RBCs to selectively adhere to the interior surface of the one or more microfluidic channels 104. In some embodiments, the coating may be Laminin or p-selectin. It may be noted that the one or more microfluidic channels 104 may be placed at any other position of the housing 204 as well, without departing from the scope of the disclosure.

Further, the at least one imaging device 106 may comprise the at least one imager 108 and the at least one processor 110. Further, the at least one imager 108 may be configured to generate one or more digital holography images or videos (not shown) of the plurality of diseased RBCs adhered to the interior surface of the one or more microfluidic channels 104. Further, the at least one imager 108 may be referred to as a digital holographic imager. In some embodiments, the at least one imager 108 may use a lensless in-line digital holography configuration to generate the one or more digital holography images or videos.

In some embodiments, the at least one imaging device 106 may comprise an illumination source 208. The illumination source 208 may be directed towards the one or more microfluidic channels 104. The illumination source 208 may be configured to facilitate the at least one imager 108 to generate the one or more digital holography images or videos. In some embodiments, the illumination source 208 may comprise a height adjustable light tube 210 and a laser driver 212 that may be installed above the at least one imager 108. The height adjustable light tube 210 may be a telescopic structure with adjustment of height from the one or more microfluidic channels 104 placed on the cross hair 202 of the housing 204.

Further, the at least one imaging device 106 may be provided with a switch 214 to control the laser driver 212 and thereby, the illumination source 208. In some embodiments, the illumination source 208 may be positioned at a height, e.g., 8 centimeters (cm) from the device 102, such that the plurality of RBCs inside the one or more microfluidic channels 104 may receive a uniform laser beam.

Further, the at least one processor 110 may be communicatively coupled to the at least one imager 108. The at least one processor 110 may be configured to receive the one or more digital holography images or videos. Further, the at least one processor 110 may analyze the generated one or more digital holography images or videos to quantify adhesion of the plurality of RBCs to the interior surface of the one or more microfluidic channels 104. In some embodiments, the adhesion of the plurality of RBCs may be quantified to generate a score or relative indicator of a user's health status and indicate the efficacy of a medical treatment of the user based on a severity of the hematologic disease in the plurality of RBCs. In various embodiments, the number of adhered diseased RBCs may be compared to a known range of values. The known range of values may be obtained from a variety of blood samples. Therefore, a relative score may be indicated by evaluating the number of adhered diseased RBCs with respect to a relevant population of samples. In some embodiments, the relative score may correspond to a relative severity of the diseased RBCs. In some embodiments, the score may correspond to a number/count of diseased RBCs selectively adhered to the internal surface of the one or more microfluidic channels 104. For example, the severity of the hematologic disease such as sickle cell disease is determined based on an approximate range of adhesion, i.e., upper range for adhesion ~2000 cells/mm^2. In some embodiments, the hematologic disease may correspond to diseases relating to surface properties of red blood cells. In some embodiments, the hematologic disease may correspond to sickle cell disease. In one example embodiment, the score or relative indicator may include one or more reference ranges to allow for quick indication of the severity of the hematologic disease. The increase in the score or relative indicator, which may be a progression through one or more reference ranges, may indicate or determine a greater severity of the hematologic disease. In some embodiments, the score may include one or more reference ranges to allow for quick indication of the severity of hematologic disease.

In some embodiments, the at least one processor 110 may be configured to analyze the generated one or more digital holography images or videos using the Artificial Intelligence/Machine Learning (AI/ML) module 114. In some embodiments, the generated one or more digital holographic images or videos may be analyzed to count the plurality of diseased RBCs that are adhered to the interior surface of the one or more microfluidic channels 104. It may be noted that the count indicates the severity of the hematologic disease in the plurality of RBCs.

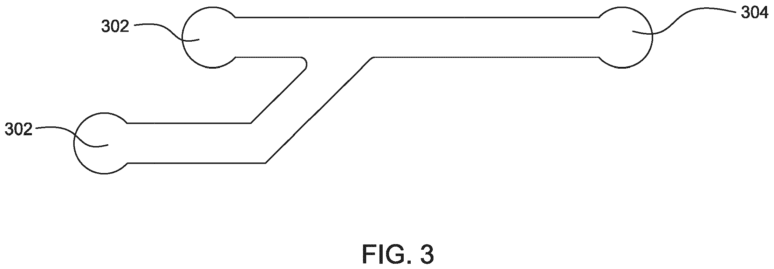

FIG. 3 illustrates the device 102 with the microfluidic channel 104 having multiple dimensions at various points in accordance with an example embodiment of the present disclosure. FIG. 3 is described in conjunction with FIGS. 1, 2A, and 2B.

The one or more microfluidic channels 104 of the device 102 may comprise at least two inlets 302 and an outlet 304. At least one inlet of the at least two inlets 302 may be configured to receive the blood sample (or red cell suspension) and the outlet 304 may be configured to facilitate discharge the plurality of RBCs that are not adhered to the interior surface of the one or more microfluidic channels 104. In some embodiments, at least one inlet of the at least two inlets 302 may be configured to receive a buffer solution to flush the plurality of RBCs that are not adhered to the interior surface of the one or more microfluidic channels 104 through the outlet 304. It may be noted that the buffer solution may be used to flush or drain the blood samples that are healthy and do not adhere to the interior surface of the one or more microfluidic channels 104.

Further, the device 102 with the one or more microfluidic channels 104 may be machined into or from a polycarbonate sheet. In some embodiments, the device 102 with the one or more microfluidic channels 104 may be integrated into the system 100. Alternatively, or additionally, in some embodiments, the device 102 may be separate from the imaging device, such as on a slide or the like that may be inserted into and removed from the imaging device. In some embodiments the device 102 may be disposable or reusable. In some embodiments, the one or more microfluidic channels 104 may have dimensions in micrometers (µm). It may be noted that the interior surface of the one or more microfluidic channels 104 may be coated so that the plurality of diseased RBCs adheres to the interior surface of the one or more microfluidic channels 104. In some embodiments, the interior surface of the one or more microfluidic channels 104 may be coated using Laminin or p-selectin. In some example embodiments, various other coating options may exist depending on which pharma medication is being evaluated for efficacy. In some example embodiments, a microfluidic channel has two inlets with dimensions of length and width as 9800 µm and 1000 µm respectively, one outlet with dimension of length and width as 3500 µm and 1000 µm respectively, and height of 50 µm. It will be apparent to one skilled in the art that the above-mentioned dimensions have been provided only for illustration purposes, without departing from the scope of the disclosure.

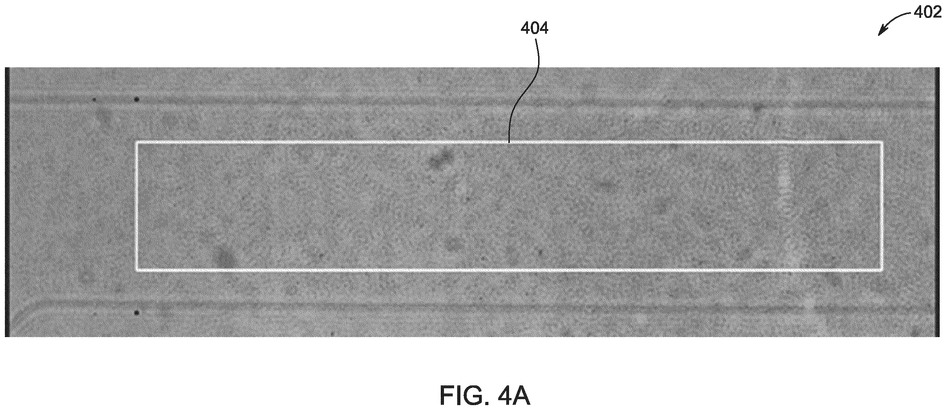

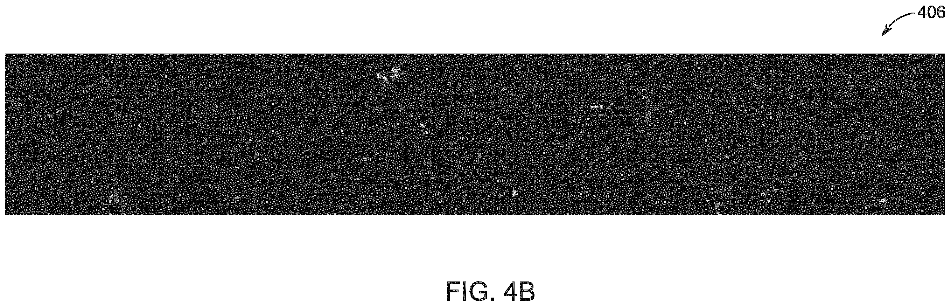

FIGS. 4A illustrates an example holographic microscopy image 402 with cell counting area 404, in accordance with an example embodiment of the present disclosure. FIG. 4B illustrates a binarized image 406 with diseased red blood cells adhering to interior surface of one or more microfluidic channels 104 in accordance with an example embodiment of the present disclosure. The blood sample cells or the plurality of diseased RBCs may selectively adhere to the coating on the interior surface of the one or more microfluidic channels 104. It may be noted that some diseased red blood cells of the plurality of RBCs adhere to the coating as the illumination source 208 illuminates the interior surface of the one or more microfluidic channels 104. In some embodiments, the cell counting area 404 may be used to determine quantity of adhesion of the plurality of RBCs per square millimeter of the interior surface of the one or more microfluidic channels 104. For example, 1 mm² of the interior surface of a microfluidic channel is adhered with 2000 cells.

As illustrated in FIG. 4B, the binarized image 406 with diseased red blood cells have adhered to the interior surface of the one or more microfluidic channels 104. The binarized image 406 may be a binarized image of the cell counting area 404 of the holographic microscopy image 402 of FIG. 4A. The digital holographic image 402 may be generated by the at least one imager 108. The digital holographic image 402 may be analyzed to automatically count the plurality of diseased RBCs that adhere to the coated interior surface of the one or more microfluidic channels 104. It may be noted that the output number of adhered diseased red blood cells may be an indication of SCD disease severity. In some embodiments, the quantification of the plurality of RBCs are configured to generate the score or relative indicator of the user's health status and/or indicate an efficacy of a medical treatment of the user based on a severity of the hematologic disease in the plurality of RBCs. In some embodiments, the plurality of RBCs may be unstained, untagged, and unmodified.

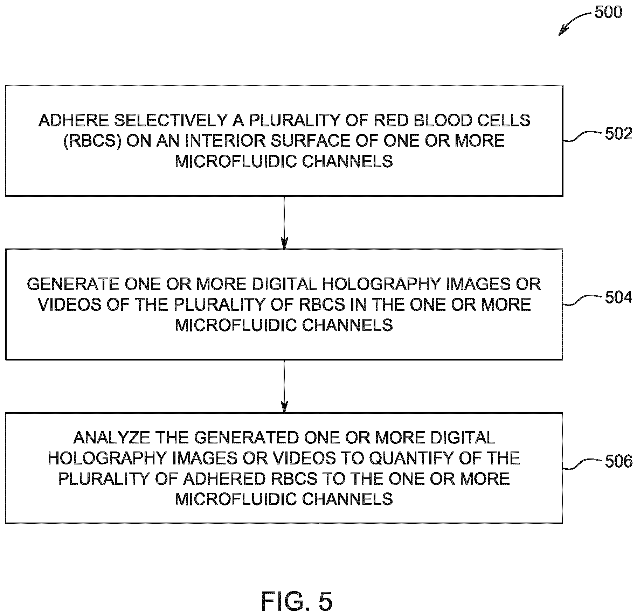

FIG. 5 illustrates a flowchart of a method 500 to determine severity of the hematologic disease i.e., the sickle cell disease, in accordance with an example embodiment of the present disclosure. FIG. 5 is described in conjunction FIGS. 1-4B.

At operation 502, the plurality of RBCs adheres on the interior surface of the one or more microfluidic channels 104. As discussed above, the interior surface of the one or more microfluidic channels 104 may be coated and the diseased red blood cells of the plurality of RBCs may adhere to the coating. In some embodiments, the at least one inlet of the at least two inlets 302 may receive the blood sample, i.e., plurality of RBCs, and then the buffer solution may be flown from another inlet of the at least two inlets 302 to flush the plurality of RBCs that do not adhere to the interior surface of the one or more microfluidic channels 104 from the outlet 304.

At operation 504, the at least one imager 108 generates the one or more digital holography images or videos of the plurality of diseased RBCs adhered to the interior surface of the one or more microfluidic channels 104. In some embodiments, the at least one imager 108 may use a lensless in-line digital holography configuration to generate the one or more digital holography images or videos. In some embodiments, the illumination source 208 may illuminate the one or more microfluidic channels 104 to facilitate the at least one imager to generate the one or more digital holography images or videos.

At operation 506, analyze the generated one or more digital holography images or videos to quantify adhesion of the plurality of RBCs to the interior surface of the one or more microfluidic channels 104. In some embodiments, the at least one processor 110 may be configured to analyze the generated one or more digital holography images or videos using Artificial Intelligence/Machine Learning (AI/ML) techniques. In some embodiments, the at least one processor 110 may analyze the generated one or more digital holographic images or videos to count the plurality of diseased RBCs that are adhered to the interior surface of the one or more microfluidic channels 104. In some embodiments, the adhesion of the plurality of RBCs may be quantified to generate the score or relative indicator of a user's health status and indicate the efficacy of the medical treatment of the person.

It will be appreciated that the method 500 may be implemented by one or more the embodiments disclosed herein, which may be combined or modified as desired or needed. Additionally, the steps in the method 500 may be modified, changed in order, performed differently, performed sequentially, concurrently or simultaneously, or otherwise modified as desired or needed.

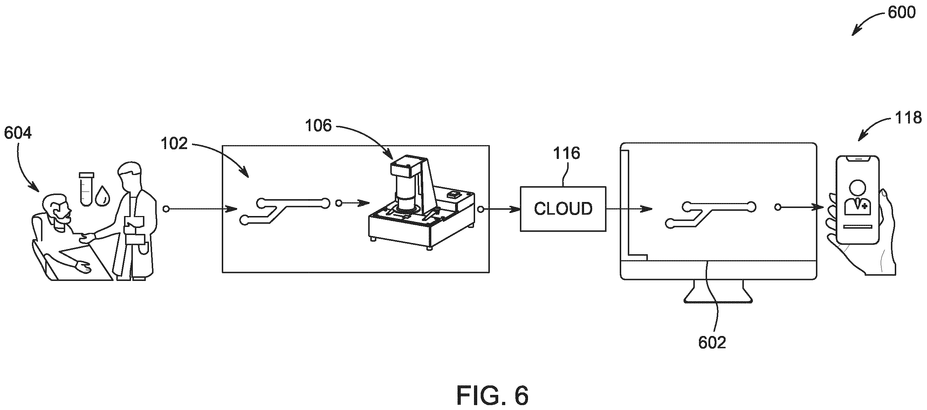

FIG. 6 illustrates an exemplary scenario 600 of the system 100 to determine severity of the sickle cell disease, in accordance with an example embodiment of the present disclosure. FIG. 6 is described in conjunction with FIGS. 1-5.

In some embodiments, the exemplary scenario 600 may comprise the at least one imaging device 106 as discussed above in conjunction with FIGS. 1-5. Further, the system 100 may comprise a display device 602 configured to display the quantified adhesion of the plurality of RBCs. Further, the system 100 may comprise the at least one user device 118 for receiving a report related to a health status of a user 604. In one embodiment, the user 604 may correspond to a patient. It may be noted that the display device 602 may correspond to a doctor's or user's console such that the doctor or user may analyze the adhesion of the diseased RBCs. The at least one user device 118 may include a smartphone, a tablet, a laptop, a personal computer (PC), a smart watch or any other device known in the art to display the report. In some embodiments, the report may comprise at least one score or relative indicator of the user 604 health status and/or indicate an efficacy of the medical treatment of the user 604 based on a severity of the sickle cell disease in the plurality of RBCs. The system 100 may further transfer the report to the user 604, via the cloud 116. In some embodiments, the AI/ML techniques may be implemented over the cloud 116 to interpret the data of the report entailing the details of the user 604 health status.

As discussed above, the at least one processor 110 may be configured to analyze the generated one or more digital holography images or videos using AI/ML techniques. The AI/ML techniques may be configured to count total adhered diseased RBCs after the plurality of RBCs that pass through the one or more microfluidic channels 104. The AI/ML techniques may implement AI/ML algorithms to quantify adhesion of the plurality of RBCs to the interior surface of the one or more microfluidic channels 104. It may be noted that the total number of the plurality of the diseased RBCs adhered to the interior surface may be a proxy measurement for the severity of the SCD. It may also be noted that the diseased RBCs adhere to the interior surface of the one or more microfluidic channels 104.

The present disclosure utilizes microfluidic channels and a digital holography technique to determine severity of the hematologic diseases, like sickle cell disease. The microfluidic channels are coated/functionalized to measure and detect the increased adhesion of diseased red blood cells relative to healthy red blood cells. The images/videos are analyzed to quantify adhesion of blood cells to the microfluidic channel surfaces. Therefore, a detailed report of severity of the sickle cell disease is provided to the user remotely. The present disclosure also provides a benefit of not labelling the cells, that is, the cells are unstained, untagged, and unmodified. This provides a cost benefit of determining the severity of diseases without using fluorescent dyes for tagging the cells.

Many modifications and other embodiments of the disclosure set forth herein will come to mind to one skilled in the art to which the present disclosure pertains having the benefit of the teachings presented in the foregoing descriptions and the associated drawings. Therefore, it is to be understood that the present disclosure is not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

## Claims

1. A system comprising:
a device comprising one or more microfluidic channels configured to adhere a plurality of diseased red blood cells (RBCs) of a blood sample on an interior surface of the one or more microfluidic channels;
at least one imager configured to generate one or more digital holography images or videos of the plurality of diseased RBCs adhered to the interior surface of the one or more microfluidic channels; and
at least one processor operationally coupled to the at least one imager and configured to:
receive the one or more digital holography images or videos; and
analyze the generated one or more digital holography images or videos to quantify adhesion of the plurality of diseased RBCs to the interior surface of the one or more microfluidic channels.

2. The system of claim 1, wherein the interior surface of the one or more microfluidic channels is coated with a coating to cause the plurality of diseased RBCs to adhere to the interior surface of the one or more microfluidic channels.

3. The system of claim 2, wherein the coating comprises at least one of Laminin or p-selectin.

4. The system of claim 1, wherein the at least one imager uses a lensless in-line digital holography configuration to generate the one or more digital holography images or videos.

5. The system of claim 1, wherein the at least one processor is configured to analyze the generated one or more digital holography images or videos using an Artificial Intelligence/Machine Learning (AI/ML) module to determine a number of adhered RBCs of the plurality of diseased RBCs on the interior surface of the one or more microfluidic channels.

6. The system of claim 1, wherein a quantification of the plurality of RBCs adhered is configured to generate a score or relative indicator of a user's health status or indicate an efficacy of a medical treatment.

7. The system of claim 1, wherein the plurality of diseased RBCs are unstained, untagged and unmodified.

8. The system of claim 6, wherein at least one user device is configured to receive the quantified adhesion of the plurality of diseased RBCs to display a report related to the user's health status.

9. A method comprising:
adhering a plurality of diseased red blood cells (RBCs) of a blood sample on an interior surface of one or more microfluidic channels;
generating, via at least one imager, one or more digital holography images or videos of the plurality of RBCs adhered to the interior surface of the one or more microfluidic channels; and
analyzing, via at least one processor, the generated one or more digital holography images or videos to quantify adhesion of the plurality of RBCs to the interior surface of the one or more microfluidic channels.

10. The method of claim 9, wherein the interior surface of the one or more microfluidic channels is coated with a coating to cause the plurality of diseased RBCs to adhere to the interior surface of the one or more microfluidic channels.

11. The method of claim 10, wherein the coating comprises at least one of Laminin or p-selectin.

12. The method of claim 9, wherein the at least one imager uses a lensless in-line digital holography configuration to generate the one or more digital holography images or videos.

13. The method of claim 9 further comprising:
analyzing, via the at least one processor, the generated one or more digital holography images or videos using an Artificial Intelligence/Machine Learning (AI/ML) module to determine a number of adhered RBCs of the plurality of diseased RBCs on the interior surface of the one or more microfluidic channels.

14. The method of claim 9, wherein a quantification of the plurality of diseased RBCs adhered is configured to generate a score or relative indicator of a user's health status or indicate an efficacy of a medical treatment.

15. The method of claim 9, wherein the plurality of diseased RBCs are unstained, untagged, and unmodified.
